Europäisches Patentamt

European Patent Office

Office européen des brevets

Veröffentlichungsnummer: **0 052 284**
**A2**

## EUROPÄISCHE PATENTANMELDUNG

Anmeldenummer: **81109240.2**

Anmeldetag: **29.10.81**

Int. Cl.³: **A 61 M 15/00**
**A 61 M 11/06**

Priorität: **17.11.80 DE 3043377**

Veröffentlichungstag der Anmeldung:
**26.05.82 Patentblatt 82/21**

Benannte Vertragsstaaten:
**CH FR GB LI SE**

Anmelder: **Brugger, Inge**
**Prinz-Karl-Strasse 50a**
**D-8130 Starnberg(DE)**

Erfinder: **Brugger, Inge**
**Prinz Karl Strasse 50a**
**D-8130 Starnberg(DE)**

Erfinder: **Steil, Emeram**
**Georg-Queriweg 4**
**D-8130 Starnberg(DE)**

Vertreter: **Eitle, Werner, Dipl.-Ing. et al,**
**Hoffmann. Eitle & Partner Patentanwälte**
**Arabellastrasse 4**
**D-8000 München 81(DE)**

Zerstäuber.

Die Erfindung betrifft einen Zerstäuber für die Vernebelung von flüssigen oder festen Stoffen, wie Medikamente, mit Hilfe eines Druckluftstromes, insbesondere zu Inhalati-onszwecken. Der Zerstäuber besitzt eine in einem Vernebe-lungsraum (10) befindliche Zerstäuberdüse (3), die an einen Druckluftkanal (2) angeschlossen ist und eine Ansaugeinrichtung zum Ansaugen des Medikamentes besitzt, wobei der Druckluftkanal neben der Düse eine außer Funktion setzbare Abblasöffnung (15) zum Entweichen der Druckluft hat, die zum Beispiel beim Einatmen des Patienten verschlossen und beim Ausatmen geöffnet werden kann. Bei vorbekannten Zerstäubern wird die durch die Abblasöffnung abgeleitete Druckluft unter zischender Geräuschentwicklung und der Gefahr der Verletzung des Bedienenden ins Freie geleitet. Um diesen Nachteil zu vermeiden, mündet bei dem erfin-dungsgemäßen Zerstäuber die Abblasöffnung in den Verne-belungsraum. Zweckmäßig liegt diese Abblasöffnung an derjenigen Seite des Druckluftkanales, die dem Aufnahme-raum (7) für den zu vernebelnden Stoff abgewandt ist.

*Fig. 1*

- 1 -

Zerstäuber:

Die Erfindung betrifft einen Zerstäuber für die Vernebelung von flüssigen oder festen Stoffen, wie Medikamente, mit Hilfe eines Druckluftstromes, insbesondere zu Inhalationszwecken, mit einer in einem Vernebelungsraum befindlichen Zerstäuberdüse, die an einen Druckluftkanal angeschlossen ist und eine Ansaugeinrichtung zum Ansaugen des Medikamentes besitzt, wobei der Druckluftkanal neben der Düse eine außer Funktion setzbare Abblasöffnung der Druckluft hat. Mit den bekannten Zerstäubern der vorgenannten Gattung werden die zu vernebelnden Stoffe nach ihrer Vernebelung in angesaugter Luft mit dieser einem Auslaß des Zerstäubergehäuses zugeführt, um,z.B. bei einem Inhalationsgerät, von dem Patienten eingeatmet zu werden. Da dem Patienten nur während seiner Einatemphase vernebeltes Medikament zugeführt werden muß, kann bei den bekannten Zerstäubern die Zerstäubung unterbrochen werden. Zu diesem Zweck besitzt bei ihnen der Druckluftkanal eine zum Entweichen der Druckluft dienende, außer Funktion setzbare bzw. verschließbare Abblasöffnung, die beim Einatmen verschlossen und beim Ausatmen geöffnet werden kann. Bei geöffneter Abblasöffnung strömt die im Druckluftkanal ankommende Druckluft durch die Abblasöffnung ab, ohne durch die Düse hindurchgedrückt zu werden. Eine Vernebelung findet also nicht statt.

Die bei dem bekannten Zerstäuber durch diese Abblasöffnung ins Freie geleitete Druckluft führt zu einem hörbaren, störenden zischenden Geräusch und läßt außerhalb einen Luftstrahl entstehen. Dabei besteht die Gefahr, daß dieser Luftstrahl auf die Hand oder ins Gesicht des inhalierenden Patienten bläst, was für diesen sehr unangenehm sein kann.

Der Erfindung liegt die Aufgabe zugrunde, die vorgenannten Nachteile bei einem Zerstäuber der eingangs genannten Gattung zu vermeiden. Dies wird erfindungsgemäß dadurch erreicht, daß die Abblasöffnung in den Vernebelungsraum mündet. Hierdurch werden ein außerhalb des Zerstäubers hörbares zischendes Luftgeräusch sowie ein an der Außenseite des Zerstäubergehäuses austretender, unangenehm in Erscheinung tretender Luftstrahl vermieden. Ferner wird hierdurch bei Anwendung des Geräts zu Inhalationszwecken der nicht unerhebliche Vorteil erreicht, daß auch während der Ausatmungsphase bei geöffneter Abblasöffnung ein etwas erhöhter Luftdruck im Vernebelungsraum bestehen bleibt, der verhindert, daß bei Ausatmen des Patienten die Ausatmungsluft in den Vernebelungsraum hineingelangt, um anschließend vom Patienten wieder eingeatmet zu werden, was eine unerwünschte Verringerung des Frischluftanteiles des nächsten Atmungszuges zur Folge hat.

Bei einer bevorzugten Ausführungsform liegt die Abblasöffnung an derjenigen Seite des Druckluftkanals, die dem Aufnahmeraum für den zu vernebelnden Stoff abgewandt ist. Hierdurch wird eine unkontrollierte Verwirbelung des Stoffes beim Abblasen der Druckluft vermieden. Da sich der Aufnahmeraum für den zu vernebelnden Stoff in der Regel unterhalb des Druckluftkanals und seiner Zerstäuberdüse befindet, sollte die Ausströmöffnung bei funktionsbedingter Aufstellung des Zerstäubers mit nach oben gerichteter Düse ebenfalls nach oben in den Vernebelungsraum gerichtet sein. Sie besitzt zweckmäßig einen an der Außenseite des Druckluftkanals befindlichen stutzenartigen Kragen, der ein Eindringen von grob oder noch nicht zerstäubtem Stoff verhindert. Außerdem kann dieser Kragen dazu dienen, die entweichende Luft an eine Umlenkfläche heranzuführen, die über der Abblasöffnung im Vernebelungsraum angeordnet sein kann.

Bei einer bevorzugten Ausführungsform des erfindungsgemäßen Zerstäubers ist die Abblasöffnung in einem an der
Zerstäuberdüse vorbeiführenden Teil des Druckluftkanals
angeordnet, der zwischen Düse und Abblasöffnung durch ein
von Hand betätigbares Ventil abschließbar ist, welches
einen Ventilstift hat, der in Richtung der Achse des Kanalteiles in diesen hineinbewegbar ist.

Weitere Einzelheiten und Vorteile der Erfindung ergeben
sich aus der in der Zeichnung dargestellten besonders
zweckmäßigen Ausführungsform des erfindungsgemäßen Zerstäubers, die im Folgenden näher beschrieben wird:

Fig. 1 zeigt einen Vertikalschnitt durch diese Ausführungsform;

Fig. 2 veranschaulicht einen Vertikalschnitt durch den
Druckluftkanal im Bereich der Zerstäuberdüse und der
Abblasöffnung in größerem Maßstab;

Fig. 3 ist ein Vertikalschnitt durch die Zerstäuberdüse
und Druckluftkanal nach Linie III in Fig. 2.

Bei dem in der Zeichnung dargestellten als Inhalationsgerät ausgebildeten Ausführungsbeispiel des erfindungsgemäßen Zerstäubers ist im unteren Teil des zylindrischen Zerstäubergehäuses 1 ein an eine Druckluftquelle
anschließbarer Druckluftkanal 2 vorgesehen, der sich vom
Umfang des Zerstäubergehäuses 1 aus in das Innere des
Gehäuses bis zur Gehäuseachse und an dieser vorbei erstreckt. Im Bereich der Gehäuseachse ist an den Druckluftkanal die Zerstäuberdüse 3 angeschlossen, die in einen Ringkanal 5 mündende Ansaugkanäle 4 besitzt, durch

- 4 -

welche der Düsenmündung die an dieser zu zerstäubenden Stoffe (Medikamente) zugeführt werden können, die über ein Saugrohr 6 aus dem unterhalb des Druckluftkanals 2 befindlichen Aufnahmeraum 7 für das zu zerstäubende Medikament 8 hineinragt.

Oberhalb der Düse befindet sich ein kaminförmiger Ansaugkanal 9, der koaxial im Zerstäubergehäuse 1 angeordnet ist und in geringem Abstand oberhalb der Düse endet. Der an der Oberseite der Düse beim Zerstäubungsvorgang entstehende Unterdruck saugt über diesen Ansaugkanal Zusatzluft an, die sich mit der aus der Düse austretenden Druckluft und dem von dieser angesaugten Medikament im Vernebelungsraum 10 zu einem homogenen Nebel 11 vermischt, der in dem zwischen dem Zerstäubergehäuse 1 und dem Ansaugkanal 9 gebildeten Ringkanal 12 entlang einer Wendelfläche 13 zum Ausstrittsstutzen 14 strömt, um dort unmittelbar oder über ein daran angeschlossenes Mundstück dem Patienten zugeführt werden zu können.

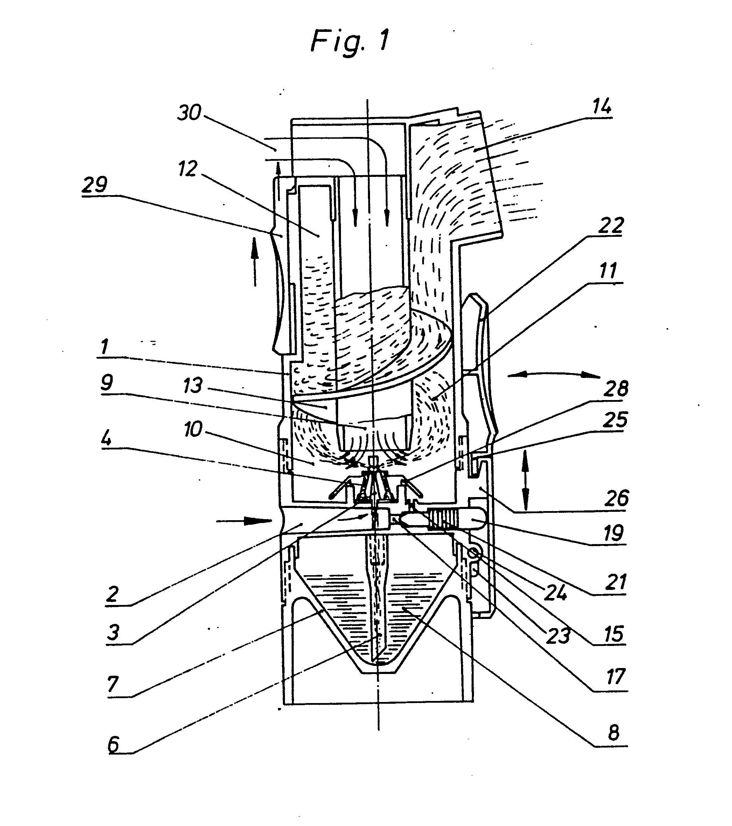

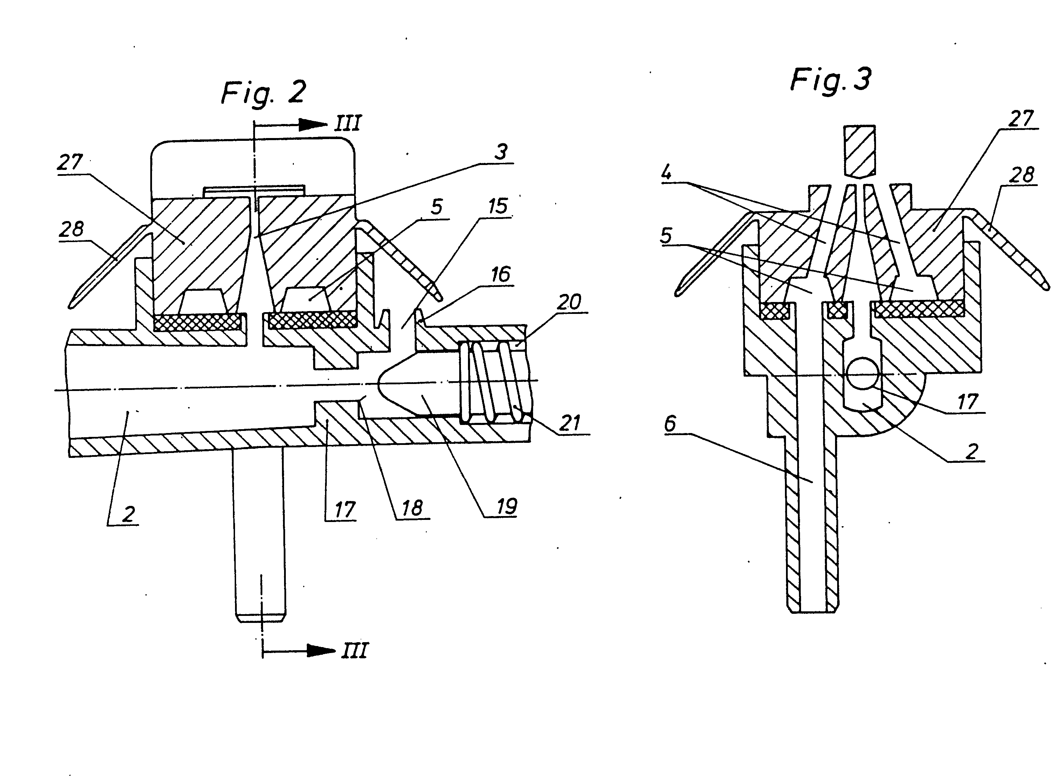

Der Druckluftkanal 2 erstreckt sich mit seinem im Zerstäubergehäuse 1 befindlichen Ende bis zu einer nach oben in den Vernebelungsraum 10 gerichteten Abblasöffnung 15, die an der Außenseite des Druckluftkanals 2 mit einem stutzenartigen Kragen 16 versehen ist. Der Druckluftkanal 2 ist zwischen dieser Abblasöffnung und der Zerstäuberdüse 3 mit einer stufenartigen Einschnürung 17 versehen, die die Anschlagfläche bzw. den Sitz 18 für einen Ventilstift 19 bildet, der in Richtung der Kanalachse aus der in Fig. 2 gezeigten zurückgefahrenen, die Abblasöffnung 15 freigebenden Stellung in den Druckluftkanal 2 bis zum Anschlag an die Ventilsitzfläche (Fig. 1) einschiebbar ist in welcher Stellung der Ventilstift die Abblasöffnung 15 verschließt. Zu diesem Zweck ist der Ventilstift in einer sich an den Druckluftkanal 2 anschlies-

- 5 -

senden Bohrung 20 im Zerstäubergehäuse 1 längsverschiebbar gelagert, in der eine Druckfeder 21 angeordnet ist, die ihn in die in Fig. 2 gezeigte zurückbewegte Stellung zu drücken sucht.

Mit seinem äußeren Ende ragt der Ventilstift aus dem Zerstäubergehäuse 1 heraus. Gegen dieses herausragende Ende liegt eine Drucktaste 22 an, die mit einem Langloch 23 auf einem am Zerstäubergehäuse 1 befestigten Achszapfen 24 sitzt. Wird die Drucktaste durch den Patienten in die in Fig. 1 gezeigte niedergedrückte Stellung bewegt, schiebt sie durch Anlage am Ventilstift 19 diesen in die ebenfalls in Fig. 1 gezeigte eingeschobene Lage, in der der Stift die Abblasöffnung 15 bzw. die Einschnürung 17 des Druckluftkanals 2 verschließt. In dieser Stellung des Ventilstiftes kann keine Druckluft durch die Abblasöffnung 15 abströmen, so daß diese gezwungen wird, durch die Düse 3 in den Vernebelungsraum 10 des Zerstäubers auszutreten, dabei über das Saugrohr 6 aus dem Medikamentenraum 7 Medikament anzusaugen und in der gleichzeitig über den Ansaugkanal 9 in den Vernebelungsraum 10 hineingesaugten Zuluft zu zerstäuben. Wird die Drucktaste freigegeben, kann der Ventilstift 19 durch die Druckfeder 21 in die in Fig. 2 gezeigte Stellung bewegt werden, in der er die Abblasöffnung 15 freigibt, durch welche sodann die Druckluft aus dem Druckluftkanal 2 entweicht. Dies führt dazu, daß die Druckluft nicht mehr durch die Düse 3 strömt, so daß in dieser Ventilstellung keine Zerstäubung stattfindet.

Um den Ventilstift in der in Fig. 1 gezeigten eingeschobenen, die Abblasöffnung 15 verschließenden Stellung festhalten zu können, ist an der dem Zerstäubergehäuse 1 zugewandten Unterseite der Drucktaste eine Anschlagnase 25 vorgesehen, die durch Verschiebung der Drucktaste mit

- 6 -

ihrem Langloch 23 auf dem Achszapfen 24 in die in Fig. 1 gezeigte untere Lagen, hinter einen als Gegenanschlag 26 wirkenden, an der Außenseite des Zerstäubergehäuses 1 angeordneten Arretierhaken bewegt werden kann, welcher die Drucktaste in niedergedrückter Lage festhält.

Am Umfang des zylindrischen Körpers 27 der Düse 3 ist ein dachförmig ausgebildeter Schirm 28 vorgesehen, der sich über die Abblasöffnung 15 hinwegerstreckt und eine Umlenkung des aus dieser austretenden Druckluftstrahles bewirkt. Hierdurch wird eine Zerteilung dieses Luftstrahles bewirkt, wodurch eine Minderung des durch das Abblasen der Druckluft innerhalb des Zerstäubers entstehende Geräusches erreicht wird.

Zur Veränderung der Ansaugmenge von Zusatzluft ist im oberen Bereich des Zerstäubers an der Außenseite seines Gehäuses ein Dosierschieber 29 derart auf- und abbewegbar gelagert, daß er über die Eintrittsöffnung 30 des Ansaugkanals 9 beliebig weit bis zur völligen Abdeckung dieser Öffnung geschoben werden kann. Hierdurch kann die Ansaugmenge der Zusatzluft eingestellt werden, wodurch die Menge und Dichte des aus dem Zerstäuber über den Austrittsstutzen 14 austretenden Nebels bemessen werden kann.

Patentansprüche

1. Zerstäuber für die Vernebelung von flüssigen oder festen Stoffen, wie Medikamente, mit Hilfe eines Druckluftstromes, insbesondere zu Inhalationszwecken, mit einer in einem Vernebelungsraum befindlichen Zerstäuberdüse, die an einen Druckluftkanal angeschlossen ist und eine Ansaugeinrichtung zum Ansaugen des Medikamentes besitzt, wobei der Druckluftkanal neben der Düse eine außer Funktion setzbare Abblasöffnung zum Entweichen der Druckluft hat, dadurch gekennzeichnet daß, die Abblasöffnung (15) in den Vernebelungsraum (10) mündet.

2. Zerstäuber nach Anspruch 1, dadurch gekennzeichnet, daß die Abblasöffnung (15) an derjenigen Seite des Druckluftkanals (2) liegt, die dem Aufnahmeraum (7) für den zu vernebelnden Stoff abgewandt ist.

3. Zerstäuber nach Anspruch 1, dadurch gekennzeichnet, daß sich über der Abblasöffnung (15) im Vernebelungsraum (10) eine Umlenkfläche für die Umlenkung der abblasenden Luft befindet.

4. Zerstäuber nach Anspruch 3, dadurch gekennzeichnet, daß die Umlenkfläche von einem Schirm (28) gebildet ist, der an der Seitenwand des neben der Abblasöffnung (15) befindlichen Düsenkörpers (27) angeordnet ist und sich über die Abblasöffnung (15) hinweg erstreckt.

5. Zerstäuber nach Anspruch 4, dadurch gekennzeichnet, daß der Schirm (28) sich um den gesamten Umfang des im Querschnitt kreisförmigen Düsenkörpers (27) herum erstreckt.

6. Zerstäuber nach einem der Ansprüche 1 bis 5, dadurch gekennzeichnet, daß die Abblasöffnung (15) einen an der Außenseite des Druckluftkanals (2) befindlichen stutzenartigen Kragen (16) hat.

7. Zerstäuber nach Anspruch 1, dadurch gekennzeichnet, daß die Abblasöffnung (15) in einem an der Zerstäuberdüse (3) vorbeiführenden Teil des Druckluftkanals (2) angeordnet ist, der zwischen Düse und Abblasöffnung durch ein von Hand betätigbares Ventil abschließbar ist.

8. Zerstäuber nach Anspruch 7, dadurch gekennzeichnet, daß das Ventil einen Ventilstift (19) hat, der in Richtung der Achse des hinter der Düse (3) befindlichen Kanalteiles in diesen hineinbewegbar ist.

9. Zerstäuber nach Anspruch 8, dadurch gekennzeichnet, daß die Abblasöffnung (15) in der Seitenwand des hinter der Düse (3) befindlichen Kanalteils angeordnet ist, und der Ventilstift (19) bis über die Abblasöffnung hinaus in diesen Kanalteil einschiebbar ist, wobei er in eingeschobener Stellung die Abblasöffnung schließt.

10. Zerstäuber nach Anspruch 8, dadurch gekennzeichnet, daß der hinter der Düse (3) liegende Kanalteil zwischen Düse und seiner Abblasöffnung (15) eine stufenförmige Erweiterung seines Innendurchmessers hat, die als Ventilsitz (18) für den Ventilstift dient.

11. Zerstäuber nach einem der Ansprüche 1 bis 10, dadurch gekennzeichnet, daß der Ventilstift (19) von einer Druckfeder (21) in die die Abblasöffnung (15) bzw. den Ventilsitz (18) freigebende Stellung zurückbewegbar ist.

12. Zerstäuber nach einem der Ansprüche 1 bis 11, dadurch gekennzeichnet, daß der Ventilstift (19) mit seinem, dem in den Druckluftkanal (2) hineinbewegbaren Ende abgewandten Ende aus dem Zerstäubergehäuse (1) herausragt, und daß an diesem Ende eine Drucktaste (22) anliegt, die an der Außenseite des Zerstäubergehäuses schwenkbar gelagert ist.

13. Zerstäuber nach Anspruch 12, dadurch gekennzeichnet, daß die Drucktaste (22) in ihrer den Ventilstift (19) in eingeschobene Lage drückenden Schwenkstellung am Zerstäubergehäuse (1) arretierbar ist.

14. Zerstäuber nach Anspruch 13, dadurch gekennzeichnet, daß zur Arretierung der Drucktaste (22) in der den Ventilstift (19) in eingeschobener Lage haltenden Stellung die Drucktaste mit einem Langloch (23) auf der am Zerstäubergehäuse (1) befestigten Schwenkachse (24) sitzt und mit diesem Langloch derart auf der Achse verschiebbar ist, daß eine Anschlagfläche an der Drucktaste hinter einen Gegenanschlag (26) am Zerstäuber gebracht werden kann.

- 1/2

## Fig. 1

# Fig. 2

# Fig. 3

0Ú52284